(19) Europäisches Patentamt
European Patent Office
Office européen des brevets

(11) **EP 4 491 132 A1**

(12) **EUROPEAN PATENT APPLICATION**
published in accordance with Art. 153(4) EPC

(43) Date of publication:
**15.01.2025 Bulletin 2025/03**

(51) International Patent Classification (IPC):
**A61B 10/00** (2006.01)  **A61B 5/026** (2006.01)
**A61B 5/145** (2006.01)  **A61B 5/20** (2006.01)

(21) Application number: **23779237.9**

(22) Date of filing: **03.03.2023**

(52) Cooperative Patent Classification (CPC):
**A61B 5/026; A61B 5/145; A61B 5/20; A61B 10/00**

(86) International application number:
**PCT/JP2023/008084**

(87) International publication number:
**WO 2023/189208 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.03.2022 JP 2022061027**

(71) Applicant: **Terumo Kabushiki Kaisha
Tokyo 151-0072 (JP)**

(72) Inventors:
• **SAWADA, Satoshi
Ashigarakami-gun, Kanagawa 259-0151 (JP)**
• **MACHIDA, Yoshihito
Ashigarakami-gun, Kanagawa 259-0151 (JP)**

(74) Representative: **Prüfer & Partner mbB
Patentanwälte · Rechtsanwälte
Sohnckestraße 12
81479 München (DE)**

(54) **PROGRAM, INFORMATION PROCESSING METHOD, AND INFORMATION PROCESSING APPARATUS**

(57)    To provide a program or the like that displays an index suitable for grasping a status of kidneys.

A program causes a computer to execute processing of acquiring urine information including urinary oxygen tension, calculating a renal status index on the basis of the urine information, and outputting the renal status index. The urine information includes a urinary output, a urine flow rate, urinary color, urine absorbance, a urinary sodium amount, or a urinary creatinine amount, in addition to the urinary oxygen tension. The renal status index is an index related to an estimated value of renal artery blood flow, the urine information includes the urinary oxygen tension and the urine flow rate, and the renal status index is calculated on the basis of the urinary oxygen tension and the urine flow rate, by using a predetermined algorithm.

FIG. 3

EP 4 491 132 A1

**Description**

Technical Field

The present invention relates to a program, an information processing method, and an information processing apparatus.

Background Art

A catheter for measurement of urinary oxygen tension in a bladder has been proposed in which a urinary catheter body and a probe that is inserted into the bladder through a urinary catheter to measure the urinary oxygen tension in the bladder are combined (Patent Literature 1).

Citation List

Patent Literature

Patent Literature 1: JP 2021-62073 A

Summary of Invention

Technical Problem

The urinary oxygen tension measured by the catheter of Patent Literature 1 is one of parameters indicating a status of kidneys. A medical worker such as a physician or a nurse comprehensively judges urinary oxygen tension and vital sign data of blood pressure or the like to grasp a status of kidneys of a patient.
However, for example, during whole body management in a surgery and in an intensive care unit (ICU), a medical worker performs various tasks simultaneously in parallel, and cannot concentrate on grasping a status of the kidneys.
In one aspect, an object is to provide a program or the like that displays an index suitable for grasping a status of kidneys.

Solution to Problem

A program causes a computer to execute processing of acquiring urine information including urinary oxygen tension, calculating a renal status index on the basis of the urine information, and outputting the renal status index.

Advantageous Effects of Invention

In one aspect, it is possible to provide a program or the like for displaying an index suitable for grasping a status of kidneys.

Brief Description of Drawings

Fig. 1 is an explanatory diagram describing a configuration of an information processing system.
Fig. 2 is a flowchart describing a flow of processing according to a program.
Fig. 3 is an example of a screen display.
Fig. 4 is an example of a screen display.
Fig. 5 is an example of a screen display.
Fig. 6 is an example of a screen display.
Fig. 7 is an example of a screen display.
Fig. 8 is an explanatory diagram describing a renal status model.
Fig. 9 is an explanatory diagram describing a treatment guideline model.
Fig. 10 is a flowchart describing a flow of processing according to a program of a third embodiment.
Fig. 11 is an example of a screen display of the third embodiment.
Fig. 12 is an example of a screen display of the third embodiment.
Fig. 13 is an explanatory diagram describing a renal status model of a fourth embodiment.

Fig. 14 is an example of a screen display of the fourth embodiment.
Fig. 15 is an example of a screen display of the fourth embodiment.
Fig. 16 is an explanatory diagram describing a drug model.
Fig. 17 is a flowchart describing a flow of processing according to a program of a fifth embodiment.
Fig. 18 is an explanatory diagram describing a configuration of an information processing system of a sixth embodiment.

Description of Embodiments

[First Embodiment]

It is known that a patient requiring a whole body management in a surgery and ICU has a high risk of having acute kidney injury (AKI) with which a renal function of the patient deteriorates in a short period of time. Because prognosis of the acute kidney injury is poor, it is desirable to grasp a status of kidneys of the patient at all times to grasp a sign of the acute kidney injury, and perform an appropriate preventive treatment before the acute kidney injury actually occurs.

A status of kidneys is evaluated on the basis of an evaluation index such as a serum creatinine level, a creatinine clearance, a glomerular filtration rate (GFR), or a urine flow rate. However, the serum creatinine level changes after about one day to three days from a kidney disorder event. Because the creatinine clearance and the glomerular filtration rate are calculated by using the serum creatinine level, similarly to the serum creatinine level, a change in the creatinine clearance and the glomerular filtration rate occurs after one day to three days after the kidney disorder. It is known that the urine flow rate may not change even after a kidney disorder.

Therefore, only with conventional evaluation indices, it is difficult to quickly find a sign of an acute kidney injury and perform a preventive treatment.

It is known that a change in renal tissue oxygen tension quickly reflects a change in a status of kidneys. However, in order to measure the renal tissue oxygen tension, it is necessary to insert an oxygen sensor into a renal tissue. Insertion of the oxygen sensor into a renal tissue has a high degree of invasion into a patient, and thus damages kidneys.

Meanwhile, a bladder-indwelling catheter is often used during whole body management in a surgery and in an ICU. Therefore, the urinary oxygen tension in the bladder can be measured in real time by using, for example, the catheter of Patent Literature 1 without increasing the degree of invasion into the patient.

The present inventors measured changes in renal tissue oxygen tension, urinary oxygen tension, urine flow rates, and pulse pressures of pigs while changing concentration of Oxygen concentration in respiratory gas during anesthesia from the pigs. The pulse pressure is a difference between a systolic blood pressure and a diastolic blood pressure. The number of samples, that is, the number of pigs used, is six.

A correlation coefficient r between the urinary oxygen tension and the renal tissue oxygen tension was calculated on the basis of data from the measurement, and the correlation coefficient r was 0.6581. The value of the calculated correlation coefficient r was equivalent to a correlation coefficient r in a prior art document, and it was confirmed that appropriate measurement was performed.

Results of three multiple regression analyses using the measurement data obtained in this experiment are shown in Table 1.

[Table 1]

| No. | Explanatory variable | Objective variable | Determination coefficient $R^2$ |
|---|---|---|---|
| 1 | Urinary oxygen tension Urine flow rate | Renal medullary oxygen tension | 0.627 |
| 2 | Urinary oxygen tension Pulse pressure | Renal medullary oxygen tension | 0.625 |
| 3 | Urinary oxygen tension Urine flow rate Pulse pressure | Renal medullary oxygen tension | 0.774 |

Renal medulla is a type of renal tissue and is a portion located inside a kidney. Thus, renal medullary oxygen tension is an exemplification of renal tissue oxygen tension. It can be seen from Table 1 that the renal medullary oxygen tension, which is an objective variable, can be estimated with high accuracy by using a multiple regression equation that is calculated by using, as explanatory variables, three data of the urinary oxygen tension, urine flow rate, and pulse pressure.

Furthermore, it is also found from Table 1 that, by using the multiple regression equation in which the urinary oxygen tension and the urine flow rate are used as explanatory variables, the renal medullary oxygen tension, which is the objective variable, can be estimated with high accuracy that is equal to or more than accuracy of the multiple regression equation in which the urinary oxygen tension and the pulse pressure are used as explanatory variables.

Methods for calculating the explanatory variables will be described. The urinary oxygen tension can be measured in real time by inserting the oxygen sensor into the bladder through the bladder-indwelling catheter as described above. The oxygen sensor may be disposed in a middle of the bladder-indwelling catheter, or in a middle between a terminal of the bladder-indwelling catheter and an opening of a urine bag connected by a tube from the terminal of the catheter.

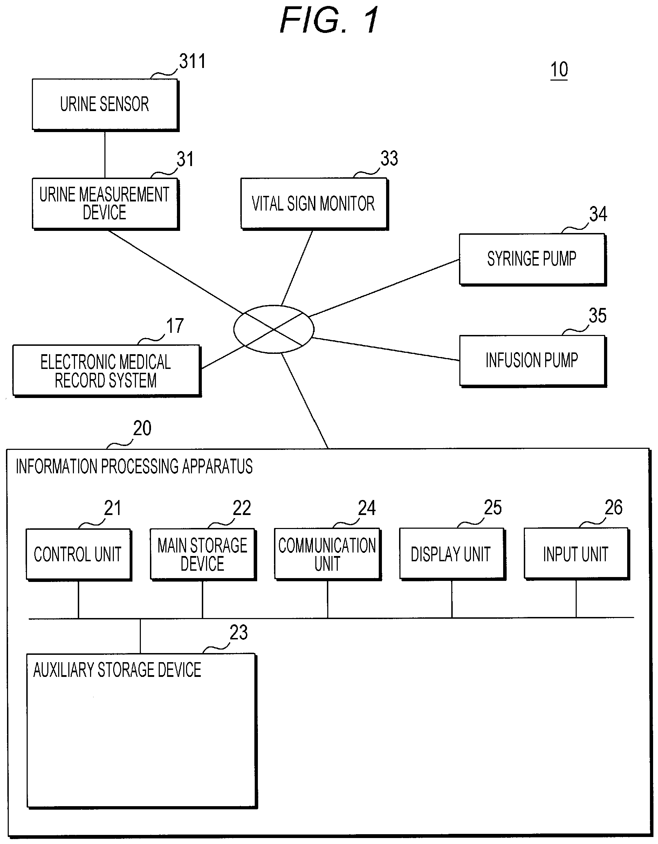

The urine flow rate can be measured in real time on the basis of, for example, a change in weight of the urine bag. The bladder-indwelling catheter may be provided with a flow sensor. A mode of the sensor for measuring the urine flow rate is not limited. During whole body management in a surgery and in an ICU, a patient is usually monitored by a vital sign monitor 33 at all times for circulatory dynamics information including blood pressure and heart rate (refer to Fig. 1).

As described above, by using the multiple regression equation, it is possible to estimate the renal medullary oxygen tension in real time with high accuracy without increasing the degree of invasion into the patient.

The renal medullary oxygen tension greatly varies among individuals depending on the circulatory dynamics and management status of the patient. Therefore, it is appropriate to judge a sign of a change in renal medullary oxygen tension leading to acute kidney injury, on the basis of a change from an initial state, for example, at a start of surgery, at a time of entering the ICU, or the like, in addition to on the basis of an absolute value of the renal medullary oxygen tension.

In the present embodiment, an index related to an estimated value of the renal medullary oxygen tension is displayed by using a relative value with an initial value at the start of surgery, at the time of entering the ICU, or the like, set to 100. That is, the display value is calculated by Mathematical Formula (1).

[Mathematical Formula 1]

$$\text{Display value} = \frac{\text{Real-time renal medullary oxygen tension}}{\text{Initial value of renal medullary oxygen tension}} \quad \text{... (1)}$$

The urinary oxygen tension and the urine flow rate are examples of urine information of the patient. The urine information may be, for example, a urinary output, urinary color, urine absorbance, a urinary sodium amount, a urinary potassium amount, or a urinary creatinine amount. The urinary output is a total amount of urine output from the bladder of the patient after the measurement is started and can be measured in real time on the basis of, for example, a weight of the urine bag.

The urinary color and urine absorbance can be measured in real time by, for example, inserting an optical fiber connected to an optical measurement device, such as a spectrophotometer, into the bladder-indwelling catheter. The urinary creatinine amount can be measured by using an enzymatic method or absorbance. In addition, the urinary sodium amount and the urinary potassium amount can be measured in real time, for example, by using a fluorescent dye that specifically reacts in a system up to the bladder-indwelling catheter and the urine bag.

The pulse pressure is an example of the circulatory dynamics information. The circulatory dynamics information may be, for example, a systolic blood pressure, a diastolic blood pressure, a mean blood pressure, a heart rate, and blood oxygen saturation. The mean blood pressure is a value calculated by diastolic blood pressure + (systolic blood pressure - diastolic blood pressure)/3. However, numerical processing of, for example, a moving average at a time of calculation, or adjustment of a calculation formula may be performed, and thus it is only required to use an equivalent value.

These pieces of circulatory dynamics information can be measured by the vital sign monitor 33. The circulatory dynamics information may be tissue oxygen saturation of each part of a body. The tissue oxygen saturation of each part of a brain or body can be measured in real time in a minimally invasive manner, and is used in clinical practice as a hemodynamic monitor.

The renal medullary oxygen tension is an example of a renal status index capable of quickly detecting a change in status of the kidneys. The renal status index may be, for example, an estimated value of renal artery blood flow. In a case where the estimated value of the renal artery blood flow is used, data for which the renal artery blood flow can be used as the objective variable in the above-described experiment is acquired, and a multiple regression analysis is performed. In addition, any index capable of detecting abnormality of the kidneys can be used.

The multiple regression equation calculated by using the urinary oxygen tension and the urine flow rate as explanatory variables and by using the renal medullary oxygen tension as the objective variable as described in No. 1 in Table 1 is an example of an algorithm for calculating the renal status index on the basis of the urinary oxygen tension and the urine flow rate.

In the measurement using the pigs as described above, in addition to the urinary oxygen tension, the urine flow rate, and the renal medullary oxygen tension, the mean blood pressure, the pulse pressure, the heart rate, the renal artery blood flow, and the glomerular filtration rate can also be measured. The multiple regression equation can be calculated by using the urinary oxygen tension, the urine flow rate, the mean blood pressure, the pulse pressure, and the heart rate as the explanatory variables, and by using the renal artery blood flow rate as the objective variable. The multiple regression equation calculated in this manner is an example of an algorithm for calculating the renal status index related to the estimated value of the renal artery blood flow on the basis of the urinary oxygen tension, the urine flow rate, the mean blood pressure, the pulse pressure, and the heart rate.

The multiple regression equation can be calculated with the same experimental data by using the urinary oxygen tension, the urine flow rate, the mean blood pressure, the pulse pressure, and the heart rate as the explanatory variables, and by using the renal medullary oxygen tension as the objective variable. The multiple regression equation calculated in this manner is an example of an algorithm for calculating the renal status index related to the estimated value of the renal tissue oxygen tension on the basis of the urinary oxygen tension, the urine flow rate, the mean blood pressure, the pulse pressure, and the heart rate.

The multiple regression equation can be calculated by using the urinary oxygen tension and the urine flow rate as the explanatory variables, and by using the renal artery blood flow rate as the objective variable. The multiple regression equation calculated in this manner is an example of an algorithm for calculating the renal status index related to the estimated value of the renal artery blood flow on the basis of the urinary oxygen tension and the urine flow rate.

Instead of the multiple regression equation, a simple regression equation may be used. For example, the simple regression equation can be calculated by using the urinary oxygen tension or the urine flow rate as the explanatory variables, and by using the renal artery blood flow rate as the objective variable. The simple regression equation calculated in this manner is an example of an algorithm for calculating the renal status index related to the estimated value of the renal artery blood flow on the basis of either the urinary oxygen tension or the urine flow rate.

For the explanatory variables, the multiple regression equation or the simple regression equation may be calculated by using the glomerular filtration rate instead of the renal artery blood flow. The multiple regression equation or simple regression equation calculated in this manner is an example of an algorithm for calculating the renal status index related to the estimated value of the glomerular filtration rate.

In addition, an algorithm for calculating an arbitrary index can be generated by calculating the multiple regression equation using a combination of arbitrary measured parameters.

A table showing a relationship between the explanatory variable and the objective variable on the basis of the multiple regression equation may be created and recorded in an auxiliary storage device 23 (refer to Fig. 1). In a case where the explanatory variables are two of the urinary oxygen tension and the urine flow rate, and the objective variable is the renal artery blood flow rate, a three-dimensional table is created. A six-dimensional table is created in a case where the explanatory variables are five of the urinary oxygen tension, the urine flow rate, the mean blood pressure, the pulse pressure, and the heart rate, and where the objective variable is the renal medullary oxygen tension.

A control unit 21 (refer to Fig. 1) searches the table by using the explanatory variables measured in real time as keys, and acquires the objective variable. The control unit 21 may calculate the objective variable by performing interpolation between the parameters recorded in the table with a known method. The table is an example of an algorithm for calculating the objective variable on the basis of the explanatory variables.

In the following description, there will be described a case, as an example, where the renal status index is the renal medullary oxygen tension, and the renal medullary oxygen tension is estimated on the basis of the urinary oxygen tension, the urine flow rate, and the pulse pressure.

Fig. 1 is an explanatory diagram describing a configuration of an information processing system 10. The information processing system 10 includes, for example, an information processing apparatus 20, a urine measurement device 31, the vital sign monitor 33, a syringe pump 34, an infusion pump 35, and an electronic medical record system 17 that are connected via a network such as a hospital information system (HIS).

The urine measurement device 31 measures and outputs urine information such as urinary oxygen tension and a urine flow rate by using a urine sensor 311. The vital sign monitor 33 is connected to various sensors (not illustrated), and measures and outputs the circulatory dynamics information such as blood pressure or pulse pressure. The urine measurement device 31 and the vital sign monitor 33 may be configured to be integrated with each other.

The syringe pump 34 is used for drug administration to the patient. The infusion pump 35 is used for infusion administration to the patient. The infusion pump 35 may have a function of mixing a drug with infusion. Instead of using the syringe pump 34, a nurse or the like may administer a drug to the patient by using a syringe. The infusion pump 35 may not be connected to the network, and may be manually operated by the nurse or the like. The syringe pump 34 and the infusion pump 35 are examples of a drug administration device.

The electronic medical record system 17 records patient information such as age, sex, height, weight, a biochemical test result, a medical history, or a course of ongoing treatment, of the patient. The course of ongoing treatment includes a type and amount of drug administered to the patient. The medical history includes the type and amount of the drug administered to the patient in a past surgery and treatment, a condition of the patient after the administration, and the like. Data such as a renal status index to be described later may be sequentially recorded in the electronic medical record system 17.

The information processing apparatus 20 includes the control unit 21, a main storage device 22, the auxiliary storage device 23, a communication unit 24, a display unit 25, an input unit 26, and a bus. The control unit 21 is an arithmetic control device that executes a program of the present embodiment. For the control unit 21, one or a plurality of central processing units (CPUs), graphics processing units (GPUs), tensor processing units (TPUs), a multi-core CPU, or the like, is used. The control unit 21 is connected to each of hardware components that constitute the information processing apparatus 20 via the bus.

The main storage device 22 is a storage device such as a static random access memory (SRAM), a dynamic random access memory (DRAM), or a flash memory. The main storage device 22 temporarily saves necessary information in the middle of processing performed by the control unit 21 and a program being executed by the control unit 21.

The auxiliary storage device 23 is a storage device such as an SRAM, a flash memory, a hard disk, or a magnetic tape. The auxiliary storage device 23 saves a program to be executed by the control unit 21 and various kinds of data necessary for executing the program. The communication unit 24 is an interface that performs communication between the information processing apparatus 20 and a network.

The display unit 25 is a liquid crystal display device, an organic electro-luminescence (EL) display device, or the like, for example. The input unit 26 is, for example, an input device such as a keyboard, a mouse, a trackball, or a microphone. The display unit 25 and the input unit 26 may be integrally stacked to constitute a touch panel.

The information processing apparatus 20 of the present embodiment is an information device such as a general-purpose personal computer, tablet, smartphone, or server computer. The information processing apparatus 20 may be a large computer, a virtual machine operating on a large computer, a cloud computing system, a quantum computer, a plurality of personal computers that performs distributed processing, or the like. The information processing apparatus 20 may be configured to be integrated with, for example, the urine measurement device 31, the vital sign monitor 33, or the electronic medical record system 17.

In the following description, a case where the control unit 21 performs software processing will be mainly described as an example. Processing described with reference to the flowchart, and various models may be implemented by dedicated hardware.

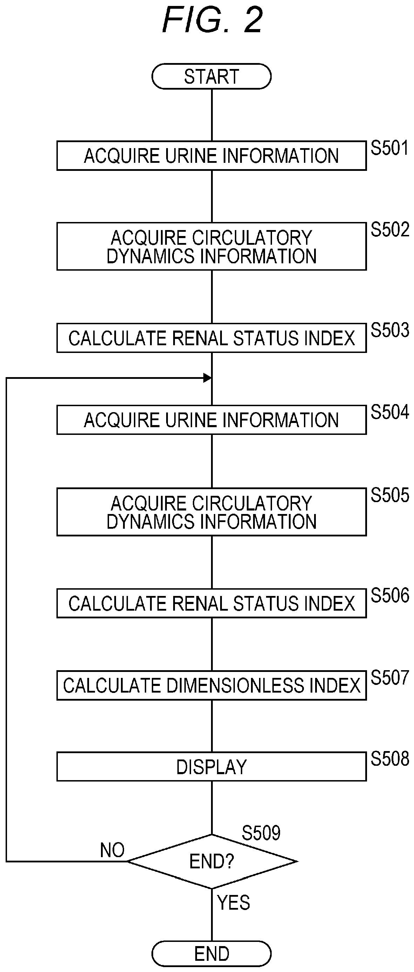

FIG. 2 is a flowchart describing a flow of processing according to a program. A user such as a physician activates the program described with reference to Fig. 2 after attaching various sensors to a body of the patient who has entered a surgery room or the ICU.

The control unit 21 acquires the urine information from the urine measurement device 31 (step S501). The control unit 21 acquires the circulatory dynamics information from the vital sign monitor 33 (step S502). The control unit 21 calculates the renal status index by substituting the urine information acquired in step S501 and the circulatory dynamics information acquired in step S502 into the multiple regression equation calculated in advance (step S503). The renal status index calculated in step S503 is an initial value of the renal status index.

The control unit 21 acquires the urine information from the urine measurement device 31 (step S504). The control unit 21 acquires the circulatory dynamics information from the vital sign monitor 33 (step S505). The control unit 21 calculates the renal status index by substituting the urine information acquired in step S504 and the circulatory dynamics information acquired in step S505 into the multiple regression equation calculated in advance (step S506). The renal status index calculated in step S506 is a real-time renal status index.

The control unit 21 calculates a dimensionless renal status index by multiplying a value, which is obtained by dividing the real-time renal status index acquired in step S506 by the initial value of the renal status index acquired in step S503, by 100 (step S507). A dimensionless index at a start of the measurement is 100. Note that in a case where the real-time renal status index exceeds the initial value, the dimensionless index is a value exceeding 100.

The control unit 21 displays the calculated renal status index on the display unit 25 (step S508). The control unit 21 may output the renal status index to another device connected to an output device, such as the vital sign monitor 33 or the electronic medical record system 17.

The control unit 21 determines whether or not to end the processing (step S509). For example, in a case where an end instruction is received from the user, the control unit 21 determines to end the processing. The control unit 21 may determine to end the processing when the urine information and the circulatory dynamics information cannot be acquired from the urine measurement device 31 and the vital sign monitor 33.

If the control unit 21 determines not to end the processing (NO in step S509), the control unit 21 returns to step S504. If the control unit 21 determines to end the processing (YES in step S509), the control unit 21 ends the processing.

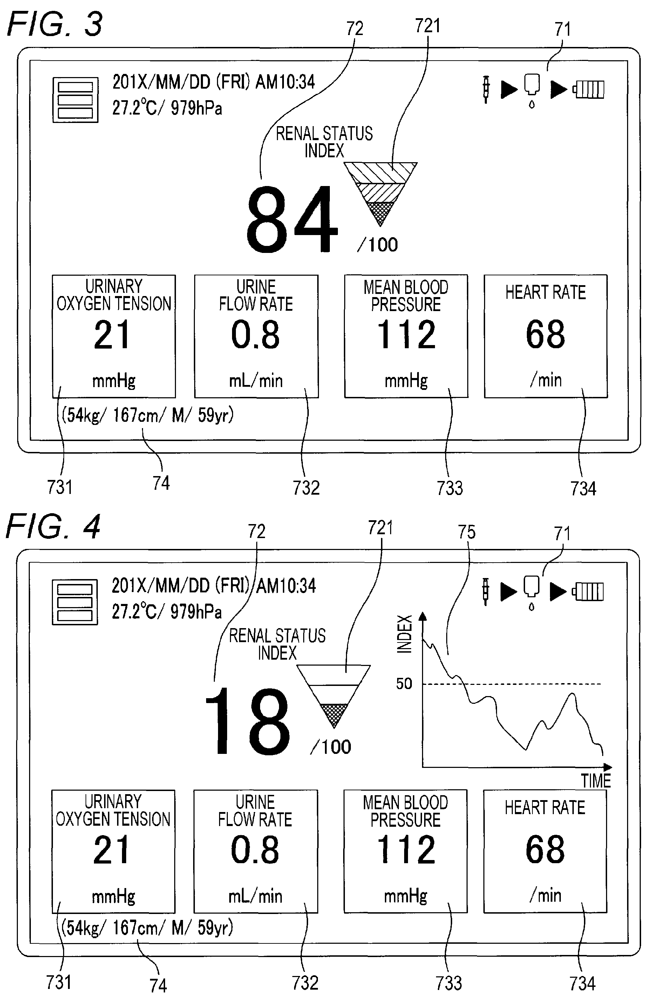

Figs. 3 to 6 are examples of a screen. The control unit 21 displays the screens illustrated in Figs. 3 to 6 in step S508 of the program described with reference to Fig. 2. The control unit 21 may display the screens illustrated in Figs. 3 to 6 on a display device disposed in, for example, a nurse station, or the like. The control unit 21 may receive selection of a screen to be displayed from the user.

The screen illustrated in Fig. 3 has a renal status index field 72, a connection state field 71, a urinary oxygen tension field 731, a urine flow rate field 732, a mean blood pressure field 733, a heart rate field 734, and a patient information field 74. A menu button is disposed in the upper left of the screen, and an indicator indicating a state of charge of a battery is disposed in the upper right of the screen. A date, day of the week, time, air temperature, and atmospheric pressure are displayed to the right side of the menu button. The air temperature and the air pressure are acquired from, for example, a sensor module that is connected to the information processing apparatus 20 and is capable of acquiring temperature and atmospheric pressure. The air temperature and the air pressure may be acquired from a world wide web (WWW) site that provides weather information via a network.

The renal status index field 72 is disposed at the center of the screen, and the dimensionless index calculated in step S507 is displayed in real time. A renal status indicator 721 is disposed next to the renal status index field 72. The renal status indicator 721 has an inverted triangular shape, and illustrates the renal status index field 72 in three stages of, for example, less than 30%, 30% or more and less than 70%, and 70% or more.

In the urinary oxygen tension field 731, the urinary oxygen tension among the urine information acquired in step S501 is displayed. In the urine flow rate field 732, the urine flow rate among the urine information acquired in step S501 is displayed. In the mean blood pressure field 733, the mean blood pressure among the circulatory dynamics information acquired in step S502 is displayed. In the heart rate field 734, the heart rate among the circulatory dynamics information acquired in step S502 is displayed.

In the patient information field 74, the patient information acquired from the electronic medical record system 17 is displayed. In the example illustrated in Fig. 3, the patient weighs 54 kilograms, and is 167 centimeters tall, male, and 59 years old.

In Fig. 3, an icon indicating the syringe pump 34 and an icon indicating the infusion pump 35 are displayed in the connection state field 71. The user can confirm that the syringe pump 34 and the infusion pump 35 are operating in a usable state. For example, in a case where a plurality of syringe pumps 34 is connected to the network, the control unit 21 may display only one icon indicating a syringe pump 34 or may display the same number of icons as the number of the connected syringe pumps 34.

In the screen illustrated in Fig. 4, a renal status graph 75 is disposed to the right side of the renal status index field 72. Because the screen is similar to the screen in Fig. 3 other than the renal status graph 75, the description of the screen other than the renal status graph 75 is omitted. The horizontal axis of the renal status graph 75 represents time. The right end of the horizontal axis represents a current time. The vertical axis of the renal status graph 75 represents the dimensionless renal status index. The user can easily check a temporal change of the dimensionless index with the renal status graph 75.

The renal status graph 75 is displayed such that a line graph extends toward the right side after the start of measurement. In a case where the right end of the renal status graph 75 is reached as illustrated in Fig. 4, the control unit 21 scrolls the renal status graph 75 by one piece of data on the right side each time new data is acquired and displays latest data at the right end of the renal status graph 75. Alternatively, for example, time may be displayed in a compressed manner to display all data.

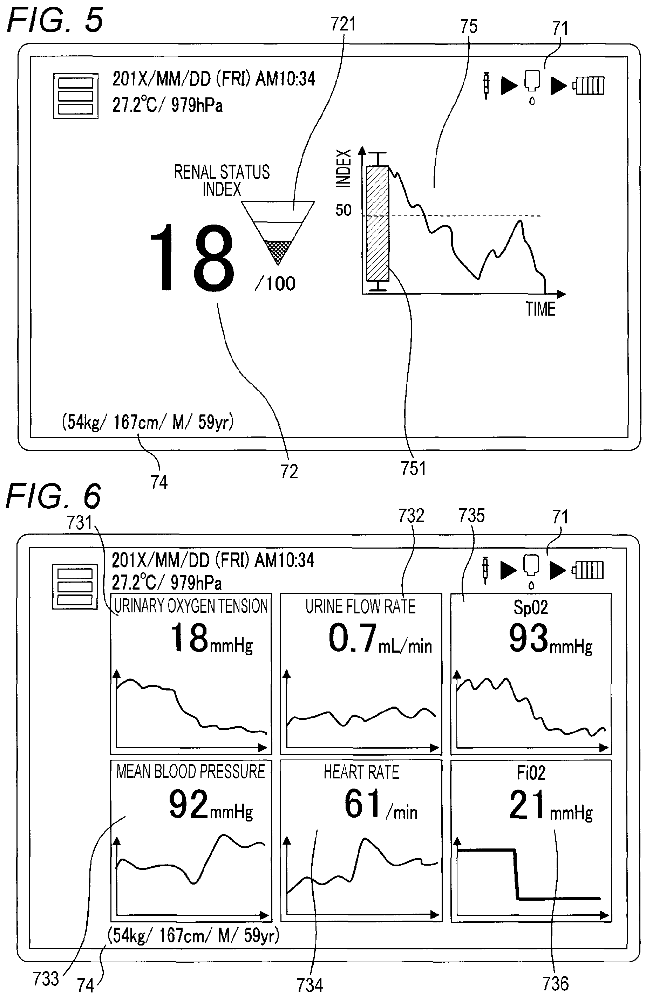

In the screen illustrated in Fig. 5, a range indicator 751 indicating a variation range of the renal status index is displayed at the left end of the renal status graph 75. The upper and lower ends of the thick portion of the range indicator 751 correspond to a maximum value and minimum value of the renal status index displayed in the renal status graph 75, respectively. Error bars indicating a calculation error of the renal status index are displayed above and below the range indicator 751. The user can easily grasp a variation range and error range of the renal status index with the range indicator 751.

In Fig. 6, the urinary oxygen tension field 731, the urine flow rate field 732, the mean blood pressure field 733, the heart rate field 734, an arterial oxygen saturation field 735, and an inhaled oxygen concentration field 736 are arranged in tiles. In the urinary oxygen tension field 731, a graph illustrating a temporal change in the urinary oxygen tension and a latest urinary oxygen tension are displayed. In the graph in the urinary oxygen tension field 731, the horizontal axis represents the time, and the vertical axis represents the urinary oxygen tension.

In the urine flow rate field 732, a graph illustrating a temporal change in the urine flow rate and a latest urine flow rate are displayed. In the graph in the urine flow rate field 732, the horizontal axis represents the time, and the vertical axis represents the urine flow rate. In the mean blood pressure field 733, a graph illustrating a temporal change in the mean blood pressure and a latest mean blood pressure are displayed. In the graph in the mean blood pressure field 733, the horizontal axis represents the time, and the vertical axis represents the mean blood pressure. In the heart rate field 734, a graph illustrating a temporal change in the heart rate and a latest heart rate are displayed. In the graph in the heart rate field 734, the horizontal axis represents the time, and the vertical axis represents the heart rate.

In the arterial oxygen saturation field 735, a graph illustrating a temporal change in arterial oxygen saturation (saturation of percutaneous oxygen: SpO2) and a latest arterial oxygen saturation are displayed. In the graph in the arterial oxygen saturation field 735, the horizontal axis represents the time, and the vertical axis represents the arterial oxygen saturation.

In the inhaled oxygen concentration field 736, a graph illustrating a temporal change in inhaled oxygen concentration (fraction of inspiratory oxygen: FiO2) and a latest inhaled oxygen concentration are displayed. In the graph in the inhaled oxygen concentration field 736, the horizontal axis represents the time, and the vertical axis represents the inhaled oxygen concentration. The inhaled oxygen concentration is calculated on the basis of a type of an oxygen administrator, such as a nasal cannula, a mask with a reservoir or a venturi mask, attached to the patient, and the oxygen flow rate. During general anesthesia, the inhaled oxygen concentration may be acquired from an anesthetic machine (not illustrated).

The horizontal axes of the six types of graphs illustrated in Fig. 6 are all the same. Therefore, instead of arranging the six graphs in tiles, the six line graphs may be displayed in one graph. The graphs illustrated in Figs. 4 to 6 may be bar charts.

The examples of screens described with reference to Figs. 3 to 6 are all examples. The control unit 21 may receive a change instruction from the user regarding items displayed on the screens and layout of each of the items.

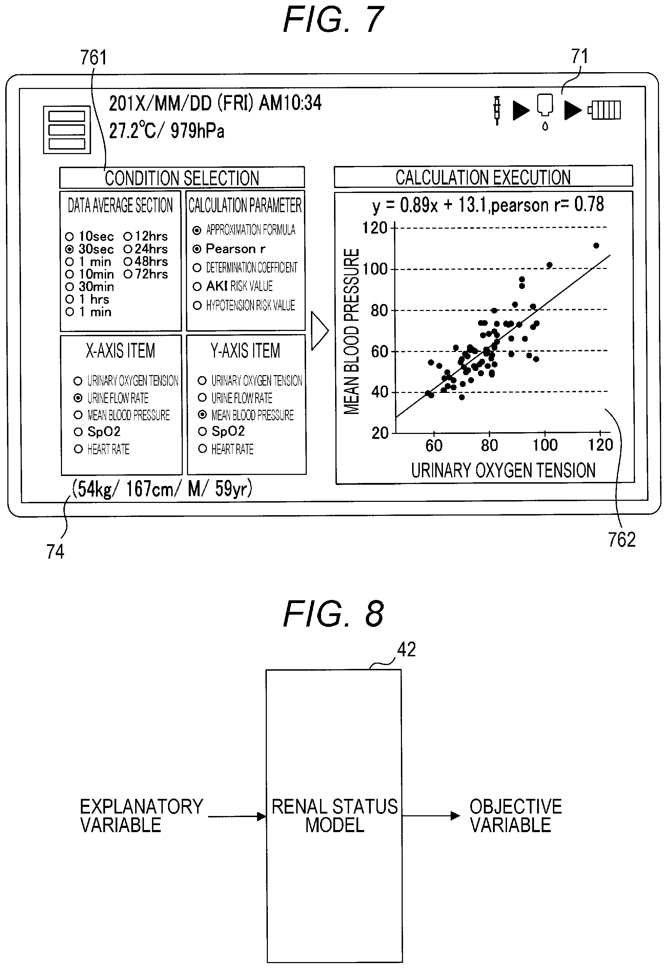

Fig. 7 is an example of a screen display. In Fig. 7, a condition selection field 761 is displayed on the left side of the screen, and a correlation graph field 762 is displayed on the right side of the screen. The horizontal axis of the graph illustrated in the correlation graph field 762 represents an item selected from "X-axis item" in the condition selection field 761. The vertical axis of the graph illustrated in the correlation graph field 762 represents an item selected from "Y-axis item" in the condition selection field 761.

The control unit 21 plots data averaged over a time selected in "data average section" in the correlation graph field 762. An item selected in "calculation parameter" is displayed in an upper part of the correlation graph field 762. In Fig. 7, an approximation formula obtained by approximating plotted data with a linear function and a Pearson's correlation coefficient are displayed. The user can check a relation between the data items by using the screen in Fig. 7.

According to the present embodiment, it is possible to provide the information processing system 10 that outputs a renal status index suitable for grasping the status of the kidneys. By displaying the dimensionless renal status, it is possible to support the user so that the user can notice a sign of acute kidney injury at an early stage, and that necessary treatment can be performed. Because it is not necessary to simultaneously grasp both the urinary oxygen tension and urine flow rate measured by the urine measurement device 31, and the pulse pressure measured by the vital sign monitor 33, it is possible to provide the information processing system 10 that reduces burden on the user. Note that, instead of the urinary oxygen tension, urinary oxygen concentration may be used.

According to the present embodiment, it is possible to provide the information processing system 10 capable of easily grasping a temporal change in the renal status index with the renal status graph 75 illustrated in Figs. 4 and 5. For example, in a case where the renal status index rapidly decreases, it can be judged that a risk of developing acute kidney injury is high. Note that, in a case where a value of the renal status index or a slope of the renal status index exceeds a predetermined threshold value, the control unit 21 may call attention of the user with sound, flashing of the display unit 25, or the like.

According to the present embodiment, it is possible to provide the information processing system 10 with which the user can simultaneously grasp a plurality of items with the screens described with reference to Fig. 6. Note that a combination of the graphs illustrated in Fig. 6 is exemplary. It is desirable that the user can select the combination of graphs to be displayed. For example, the renal status graph 75 illustrated in Figs. 4 and 5 may be one of the graphs displayed on the screen of Fig. 6.

According to the present embodiment, it is possible to provide the information processing system 10 with which the user can grasp a correlation of a plurality of data items with the screens described with reference to Fig. 7.

[Second Embodiment]

The present embodiment relates to an information processing system 10 that uses a trained model generated by machine learning instead of using a multiple regression equation. Description of portions common to the first embodiment will be omitted.

Fig. 8 is an explanatory diagram describing a renal status model 42. The renal status model 42 is a trained model that receives input of an explanatory variable and outputs an objective variable. The present inventors generated two renal status models 42 with machine learning using an XGBoost algorithm by using the experimental data described in the first embodiment as training data. The explanatory variable, objective variable, and determination coefficient of each of the renal status models 42 are shown in Table 2.

[Table 2]

| No. | Explanatory variable | Objective variable | Determination coefficient $R^2$ |
|---|---|---|---|
| 4 | Urinary oxygen tension<br>Urine flow rate | Renal medullary oxygen tension | 0.904 |

(continued)

| No. | Explanatory variable | Objective variable | Determination coefficient $R^2$ |
|---|---|---|---|
| 5 | Urinary oxygen tension<br>Urine flow rate<br>Mean blood pressure<br>Pulse pressure | Renal medullary oxygen tension | 0.915 |

Because both No. 4 and No. 5 have a determination coefficient larger than the multiple regression analyses shown in Table 1, it can be seen that renal medullary oxygen tension can be estimated more accurately than with the multiple regression analyses in shown embodiment 1 or the multiple regression equation. From No. 1 shown in Table 1 and No. 4 shown in Table 2, the renal medullary oxygen tension can be accurately estimated on the basis of two parameters of urinary oxygen tension and urine flow rate by using the renal status model 42.

XGBoost is an example of a machine learning algorithm. The trained model may be generated by using an algorithm such as random forests or convolutional neural network (CNN), for example. The renal status models 42 shown in Table 2 are exemplary.

A renal status model 42 using an explanatory variable and objective variable different from those in No. 4 and No. 5 may be generated. The explanatory variable may include patient information, such as, for example, age, sex, height, weight, a biochemical test result, a medical history, and a course of ongoing treatment, of the patient. By adding the patient information to the explanatory variable, a renal status model 42 with a highly accurate objective variable to be output can be generated.

The renal status model 42 is stored in an auxiliary storage device 23 or an external mass storage device connected to an information processing apparatus 20. In the present embodiment, in step S503 and step S506 in the flowchart described with reference to Fig. 2, a control unit 21 inputs an explanatory variable to the renal status model 42 to acquire the renal medullary oxygen tension.

For example, the control unit 21 may select and use the renal status model 42 of No. 4. After calculating each objective variable and reliability by using each of the plurality of renal status models 42, the control unit 21 may adopt an objective variable output by the renal status model 42 having high reliability. The control unit 21 may adopt representative values of a plurality of objective variables calculated by using each of the plurality of renal status models 42. As the representative values, for example, any statistical value such as an arithmetic mean value, a geometric mean value, a maximum value, or a minimum value can be used.

According to the present embodiment, it is possible to provide the information processing system 10 that accurately displays a renal status index. By using the highly accurate renal status index, it is possible to provide the information processing system 10 that displays highly reliable information regarding a state of kidneys.

[Third Embodiment]

The present embodiment relates to an information processing system 10 that displays a treatment guideline. Description of portions common to the second embodiment will be omitted.

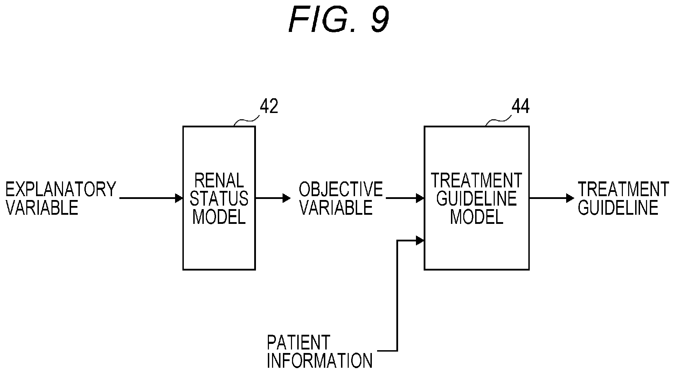

Fig. 9 is an explanatory diagram describing a treatment guideline model 44. The treatment guideline model 44 is a model that receives an objective variable output from the above-described renal status model 42 and patient information including age, sex, height, weight, a biochemical test result, and a medical history of a patient, and information related to drug administered by using a syringe pump 34 and an infusion pump 35, and outputs a treatment guideline.

As described above, the objective variable output from the renal status model 42 is a renal status index such as, for example, an estimated value of renal medullary oxygen tension or an estimated value of renal artery blood flow. The treatment guideline model 44 may receive an input of a dimensionless index obtained by non-dimensionalizing the objective variable output from the renal status model 42, with an initial value.

The renal status model 42 may receive, instead of the objective variable output from the renal status model 42, input of an objective variable calculated on the basis of the multiple regression equation described in the first embodiment.

The treatment guideline model 44 is generated by machine learning using an algorithm of random forests, XGBoost, or the like, for example. Machine learning of the treatment guideline model 44 uses training data in which a large number of sets of an objective variable and patient information, a renal status index and a treatment status, and a treatment guideline judged by a specialist are recorded. The treatment guideline is represented by, for example, a type and dosage of a drug administered by using the syringe pump 34 or the infusion pump 35.

For example, a treatment guideline model 44 that outputs a treatment guideline intended to optimize a balance between a urine flow rate and urinary oxygen is generated by using training data in which a treatment guideline or the like intended to optimize a balance between a urine flow rate and urinary oxygen is recorded. For example, a treatment guideline model 44 that outputs a treatment guideline or the like intended to bring the renal status index into a predetermined value is generated by using training data in which a treatment guideline or the like intended to bring the renal status index into a predetermined value is recorded.

The treatment guideline model 44 may be an algorithm such as a decision tree created on the basis of a rule in accordance with, for example, a guideline related to a standard treatment method defined by medical society, each medical institution, or the like.

Retraining of the treatment guideline model 44 may be performed by using a database (DB) in which treatment results of a plurality of patients are recorded. The retraining may be performed by a large computer or the like different from an information processing apparatus 20, and an updated treatment guideline model 44 may be delivered to the information processing apparatus 20 via a network. It is possible to implement the information processing system 10 that improves accuracy of the treatment guideline model 44 as needed.

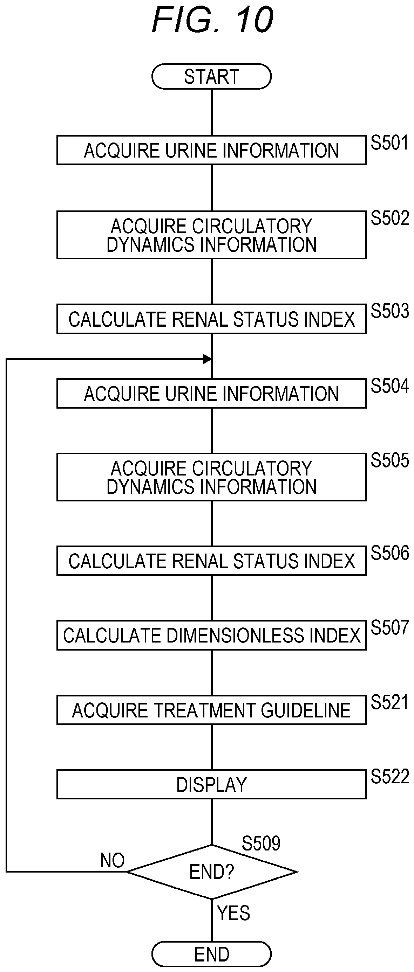

Fig. 10 is a flowchart describing a flow of processing according to a program of a third embodiment. A flow of processing up to step S507 is the same as the flow of processing of a program in the first embodiment described with reference to Fig. 2, and therefore, the description thereof is omitted.

A control unit 21 inputs, to the treatment guideline model 44, the patient information acquired from an electronic medical record system 17, the syringe pump 34, and the infusion pump 35, and the renal status index calculated in step S506 or step S507 to acquire the treatment guideline (step S521).

The control unit 21 displays the renal status index and the treatment guideline on a display unit 25 (step S522). The control unit 21 determines whether or not to end the processing (step S509). Because a subsequent processing flow is the same as the processing flow of the program according to the embodiment described with reference to Fig. 2, the description thereof will be omitted.

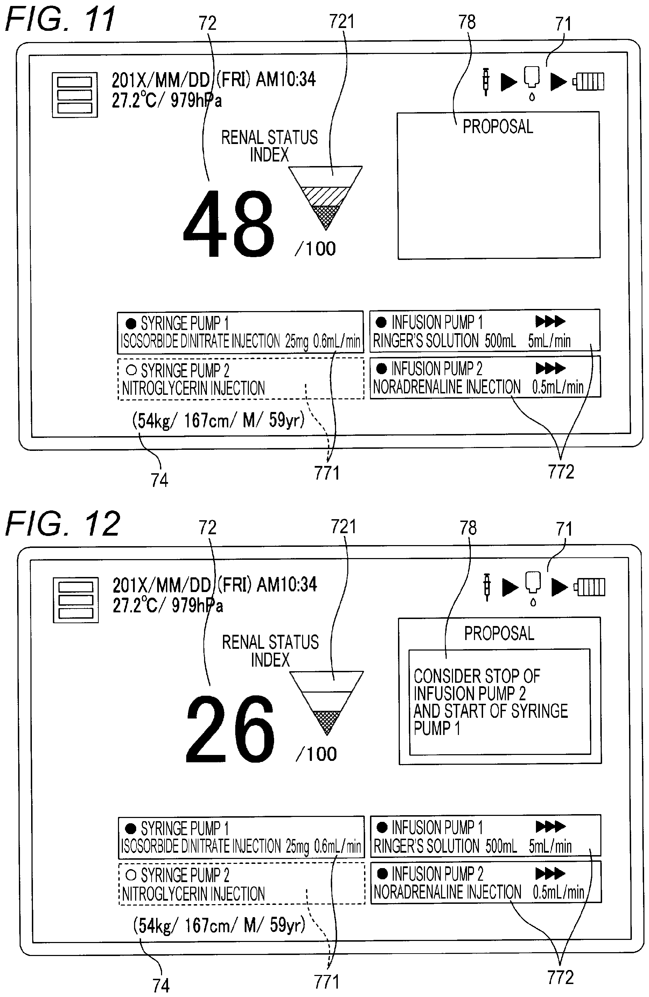

Figs. 11 and 12 are examples of a screen according to the third embodiment. On the screen illustrated in Fig. 11, in addition to a renal status index field 72, a renal status indicator 721, a connection state field 71, and a patient information field 74, two syringe pump fields 771, two infusion pump fields 772, and a proposal field 78 are arranged. In Fig. 11, there is no proposal for the treatment guideline, and the proposal field 78 is blank.

In the syringe pump fields 771, types and setting states of drugs prepared in the syringe pumps 34 are displayed. "Syringe pump 1" represents a state in which preparation for drug administration is completed. "Syringe pump 2" represents a state in which preparation for drug administration is not completed. In the infusion pump fields 772, types and administration rates of drugs prepared in the infusion pumps 35 are displayed. In Fig. 11, rightward triangles indicate that infusion is being injected into a body of the patient from "infusion pump 1" and "infusion pump 2".

In Fig. 11, there is no proposal for the treatment guideline, and the proposal field 78 is blank. In Fig. 12, a treatment guideline including two items of "stop of infusion pump 2" and "start of syringe pump 1" is displayed in the proposal field 78. According to the present embodiment, it is possible to provide the information processing system 10 that displays a treatment guideline

For example, in a case where a physician triple-taps the proposal field 78, the control unit 21 may transmit control signals to the syringe pumps 34 and the infusion pumps 35 to execute the treatment guideline displayed in the proposal field 78. It is possible to provide the information processing system 10 with which the physician can execute the displayed treatment guideline with a simple instruction.

[Fourth Embodiment]

The present embodiment relates to an information processing system 10 that predicts a change in a renal status index. Description of portions common to the second embodiment will be omitted.

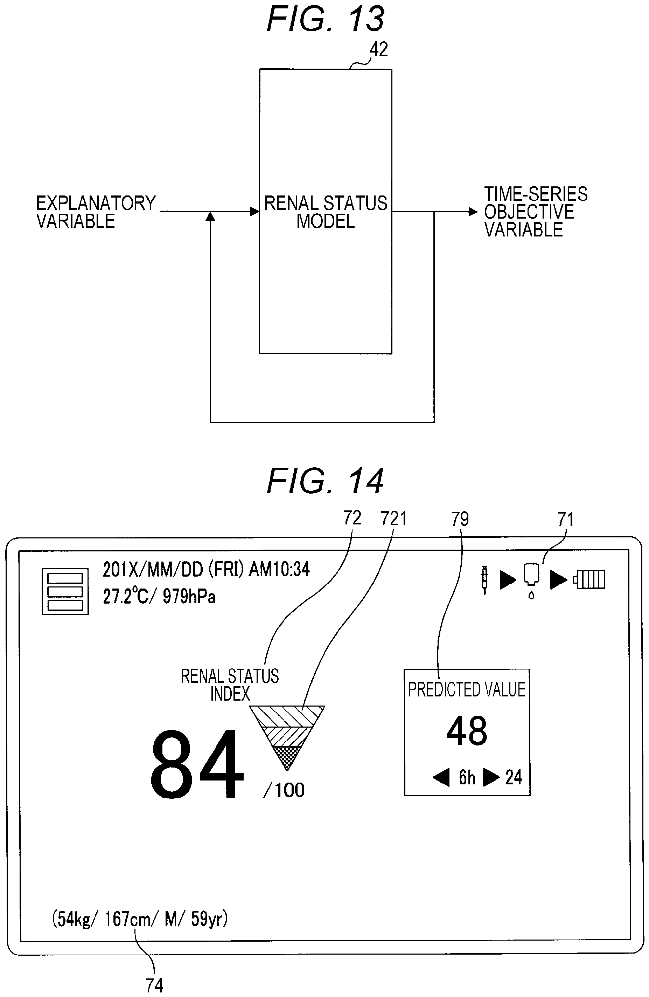

Fig. 13 is an explanatory diagram describing a renal status model 42 of a fourth embodiment. The renal status model 42 of the present embodiment receives an explanatory variable and outputs a time-series objective variable. That is, the renal status model 42 of the present embodiment outputs a predicted value related to an objective variable of a case where treatment, such as a current medication, is continued, in addition to an objective variable at the present time.

The renal status model 42 is generated by machine learning by using a training database in which a large number of sets of explanatory variables and time-series data of objective variables are recorded. The explanatory variable may include time-series data such as a time-series change in vital sign data and a time-series change in dosage. As the renal status model 42 of the present embodiment, an algorithm suitable for processing time-series data, such as, for example, a long short term memory (LSTM) or a transformer, is used. A reinforcement learning method may be used.

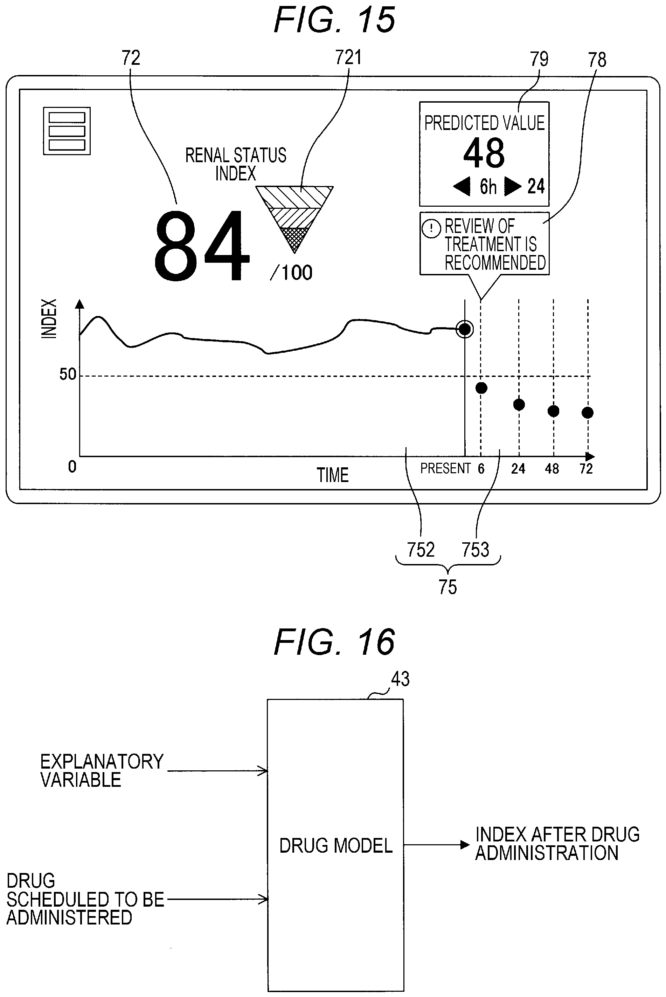

Figs. 14 and 15 are examples of a screen according to the fourth embodiment. In the screen illustrated in Fig. 14, a predicted value field 79 is disposed to the right side of a renal status index field 72. In Fig. 14, a predicted value of a renal status index after six hours is displayed in the predicted value field 79. When receiving selection of a triangle disposed on the right side of "6 h", a control unit 21 displays a predicted value for a renal status index after 24 hours.

In the screen illustrated in Fig. 15, a renal status graph 75 is disposed under the renal status index field 72 and the predicted value field 79. The horizontal axis of the renal status graph 75 represents time. The vertical axis of the renal status graph 75 represents the dimensionless renal status index.

The renal status graph 75 includes an actual value graph 752 and a predicted value graph 753. In the actual value graph 752, actual data of the renal status index is displayed. In the predicted value graph 753, predicted values of the renal status index after six hours, 24 hours, 48 hours, and 72 hours are plotted. The predicted value graph 753 has a long time-per-unit length on the horizontal axis as compare to the actual value graph 752.

Fig. 15 indicates that the renal status index decreases to nearly half of a current level after six hours if a current treatment is continued. "Review of treatment is recommended" is displayed in a proposal field 78. A user can prevent acute kidney injury by avoiding a significant change in the renal status index by changing a drug or the like.

In the present embodiment, in step S503 and step S506 in the flowchart described with reference to Fig. 2, the control unit 21 inputs an explanatory variable to the renal status model 42 described with reference to Fig. 8 to acquire time-series renal medullary oxygen tension.

According to the present embodiment, it is possible to provide the information processing system 10 that displays a predicted value of the renal status index.

[Fifth Embodiment]

The present embodiment relates to an information processing system 10 including a drug model 43 that estimates a future objective variable. Description of portions common to the first embodiment will be omitted.

Fig. 16 is an explanatory diagram describing the drug model 43. The drug model 43 is a trained model that receives an explanatory variable described with reference to Fig. 8 and the like and information related to a drug to be administered, and outputs a predicted value of an index at a time point when a predetermined time has elapsed after the drug is administered as scheduled. The index is a value that can be measured in real time, such as urinary oxygen tension, a urine flow rate, a mean blood pressure, a pulse pressure, a heart rate, or blood oxygen saturation, for example. The index may be an index that can be estimated in real time, such as an estimated value of renal artery blood flow or an estimated value of renal tissue oxygen tension. The drug model 43 may output a plurality of indices.

The drug model 43 is generated by machine learning by using, for example, training data acquired by an animal experiment using pigs, as described in the first embodiment. In a case where safety of the drug to be administered is sufficiently ensured, training data in which a large number of sets of explanatory variables related to an actual patient and an index after medication are recorded may be used. The drug model 43 may be a rule-based algorithm created on the basis of a pharmacological effect of a drug.

There are individual differences in reaction to a drug. Even if the drug model 43 generated on the basis of experimental data from pigs or data from a large number of past patients is used, it is difficult to accurately predict how each patient will react to the administered drug.

For prolonged surgery and whole body management in an ICU, a drug that affects a condition of kidneys may be administered repeatedly. By adjusting a drug dosage to the individual patient on the basis of a change in a physical condition after the drug administration, it is possible to avoid a sudden change in the condition of the patient.

For example, in a case where a change in an index after a predetermined time has elapsed is larger than a prediction output from the drug model 43 after administration of a drug according to a treatment guideline output from a treatment guideline model 44 as described with reference to Fig. 9, it can be judged that the patient is a type of patient having a strong reaction to the drug. A physician can prevent kidney injury by reducing subsequent dosage of the drug from an amount output from the treatment guideline model 44.

A control unit 21 may integrate a coefficient, which is calculated on the basis of a difference between a predicted value of an index after the drug administration, the predicted value being output from the drug model 43, and an index actually measured after a predetermined time has elapsed after the drug administration, with respect to predicted values of indices output from the drug model 43 from next time onwards, and display the predicted values. The control unit 21 may perform retraining of the drug model 43 on the basis of a difference between the predicted value of the index after the drug administration, the predicted value being output from the drug model 43, and the index actually measured after the drug administration. As described above, the information processing system 10 that outputs an index corresponding to an individual patient can be provided.

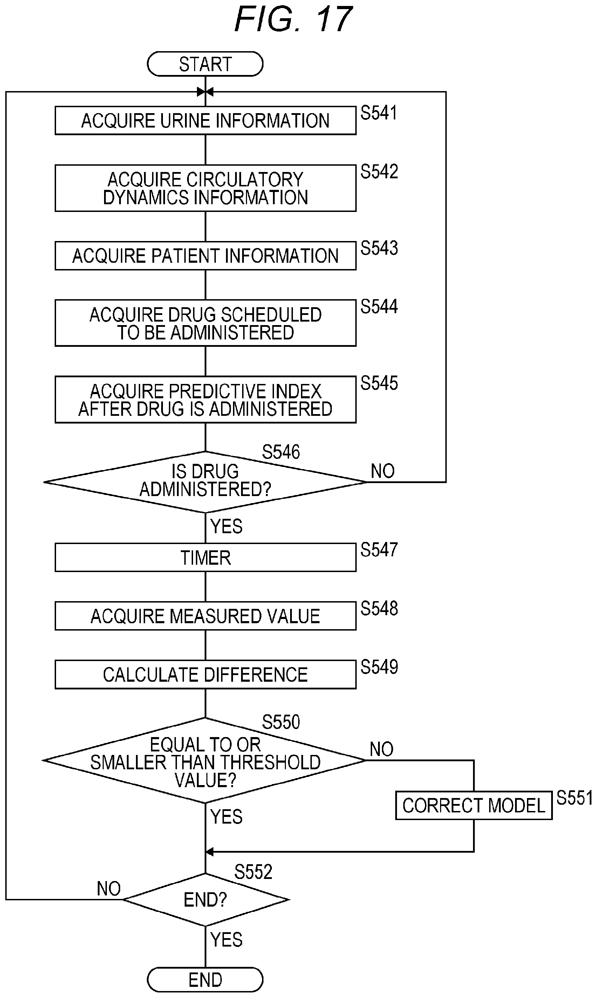

Fig. 17 is a flowchart describing a flow of processing according to a program of a fifth embodiment. The control unit 21 acquires urine information from a urine measurement device 31 (step S541). The control unit 21 acquires circulatory dynamics information from a vital sign monitor 33 (step S542). The control unit 21 acquires patient information from an electronic medical record system 17 (step S543). As described above, the patient information includes, for example, age, sex, height, weight, a biochemical test result, a medical history, and a course of ongoing treatment, of the patient.

The control unit 21 acquires information related to a drug scheduled to be administered (step S544). The information related to the drug scheduled to be administered is acquired from, for example, the treatment guideline model 44 described with reference to Fig. 9. The control unit 21 may receive input of information related to a drug scheduled to be administered by a user.

The control unit 21 inputs, to the drug model 43, the explanatory variable and information related to the drug scheduled to be administered to the patient, and acquires a predictive index at a time after the predetermined time has elapsed after the drug is administered (step S545). The control unit 21 determines whether or not the drug is administered according to a schedule acquired in step S544 (step S546). A status of the administration of the drug is acquired from, for example, a syringe pump 34 or an infusion pump 35. The control unit 21 may receive an input by the user regarding a drug administration status.

If it is determined that the drug administration as scheduled has not been performed (NO in step S546), the control unit 21 returns to step S541. If it is determined that the drug administration as scheduled has been performed (YES in step S546), the control unit 21 waits for the predetermined time for determining an effect of the drug to pass (step S547).

The control unit 21 acquires a measured value of an index on the basis of latest urine information or circulatory dynamics information (step S548). The control unit 21 calculates a difference between the predicted value acquired in step S545 and the measured value acquired in step S548 (step S549). The control unit 21 determines whether or not the difference is equal to or smaller than a predetermined threshold value (step S550).

If it is determined that the difference is not equal to or smaller than the threshold value (NO in step S550), the control unit 21 corrects the drug model 43 so that output data output from the drug model 43 approaches the measured value (step S551). Specifically, for example, the control unit 21 sets a display program so as to output a correction value obtained by integrating, with respect to the output from the drug model 43, a coefficient obtained by dividing the measured value acquired in step S548 by the predicted value acquired in step S545. The control unit 21 may receive an input by the user regarding a method for correcting the output from the drug model 43. The control unit 21 may perform retraining of the drug model 43.

If it is determined that the difference is equal to or smaller than the threshold value (YES in step S550), or after an end of step S551, the control unit 21 determines whether or not to end the processing (step S552). If the control unit 21 determines not to end the processing (NO in step S552), the control unit 21 returns to step S541. If the control unit 21 determines to end the processing (YES in step S552), the control unit 21 ends the processing.

According to the present embodiment, it is possible to provide the information processing system 10 that predicts an effect of a drug on an individual patient.

[Sixth Embodiment]

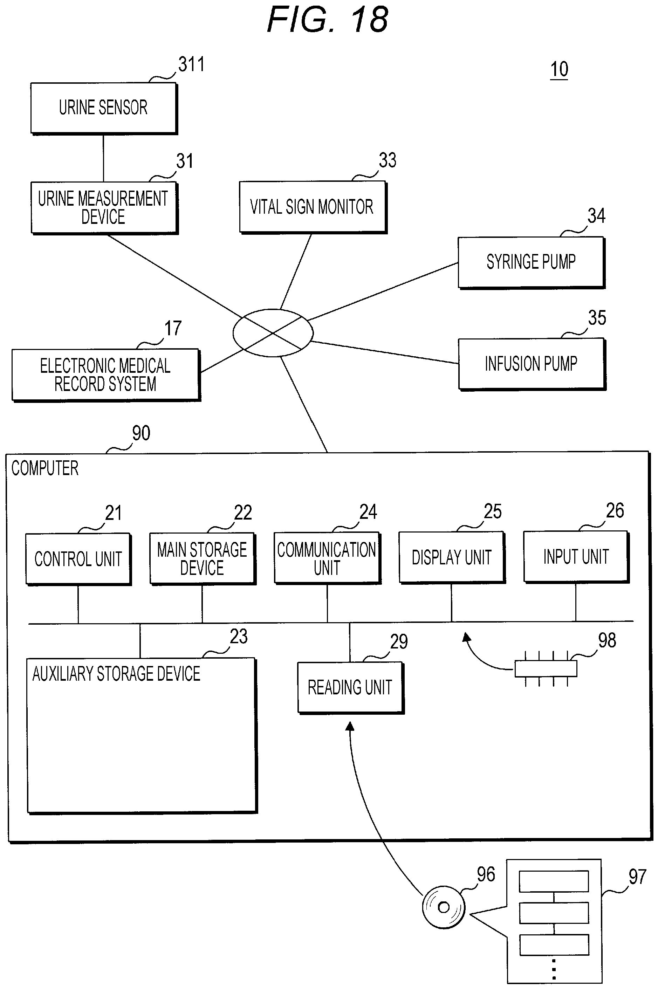

Fig. 18 is an explanatory diagram describing a configuration of an information processing system 10 of a sixth embodiment. The present embodiment relates to a mode in which an information processing apparatus 20 is implemented by causing a general-purpose computer 90 and a program 97 to operate in combination. Description of portions common to the first embodiment will be omitted.

The computer 90 includes a reading unit 29 in addition to the above-described control unit 21, main storage device 22, auxiliary storage device 23, communication unit 24, display unit 25, input unit 26, and bus.

A program 97 is recorded in a portable recording medium 96. The control unit 21 reads the program 97 via the reading unit 29 and saves the read program 97 in the auxiliary storage device 23. In addition, the control unit 21 may read the program 97 stored in a semiconductor memory 98 such as a flash memory mounted in the computer 90. Furthermore, the control unit 21 may download the program 97 from another server computer (not illustrated) connected via the communication unit 24 and a network (not illustrated) and save the downloaded program 97 in the auxiliary storage device 23.

The program 97 is installed as a control program for the computer 90 and is loaded into the main storage device 22 to be executed. As described above, the information processing apparatus 20 described in the first embodiment is implemented. The program 97 of the present embodiment is an example of a program product.

The technical features (components) described in the respective embodiments can be combined with each other, and new technical features can be formed by the combination.

It should be understood that the embodiments disclosed herein are examples in all respects and are not restrictive. The scope of the present invention is indicated not by the above meaning but by the claims, and is intended to include all changes within the meaning and scope equivalent to the claims.

Reference Signs List

10    Information processing system
17    Electronic medical record system
20    Information processing apparatus
21    Control unit

| 22 | Main storage device |
|---|---|
| 23 | Auxiliary storage device |
| 24 | Communication unit |
| 25 | Display unit |
| 26 | Input unit |
| 29 | Reading unit |
| 31 | Urine measurement device |
| 311 | Urine sensor |
| 33 | Vital sign monitor |
| 34 | Syringe pump |
| 35 | Infusion pump |
| 42 | Renal status model |
| 43 | Drug model |
| 44 | Treatment guideline model |
| 71 | Connection state field |
| 72 | Renal status index field |
| 721 | Renal status indicator |
| 731 | Urinary oxygen tension field |
| 732 | Urine flow rate field |
| 733 | Mean blood pressure field |
| 734 | Heart rate field |
| 735 | Arterial oxygen saturation field |
| 736 | Inhaled oxygen concentration field |
| 74 | Patient information field |
| 75 | Renal status graph |
| 751 | Range indicator |
| 752 | Actual value graph |
| 753 | Predicted value graph |
| 761 | Condition selection field |
| 762 | Correlation graph field |
| 771 | Syringe pump field |
| 772 | Infusion pump field |
| 78 | Proposal field |
| 79 | Predicted value field |
| 90 | Computer |
| 96 | Portable recording medium |
| 97 | Program |
| 98 | Semiconductor memory |

**Claims**

1. A program that causes a computer to execute processing comprising:

   acquiring urine information including urinary oxygen tension;
   calculating a renal status index on the basis of the urine information; and
   outputting the renal status index.

2. The program according to claim 1, wherein
   the urine information includes at least one or more of a urinary output, a urine flow rate, urinary color, urine absorbance,
   a urinary sodium amount, and a urinary creatinine amount, in addition to the urinary oxygen tension.

3. The program according to claim 1 or 2, wherein

   the renal status index is an index related to an estimated value of renal artery blood flow,
   the urine information includes at least one or more of the urinary oxygen tension and a urine flow rate, and
   the program calculates the renal status index on the basis of the urinary oxygen tension and the urine flow rate, by
   using a predetermined algorithm.

4. The program according to claim 1 or 2, wherein

   the renal status index is an index related to an estimated value of renal tissue oxygen tension,
   the urine information includes at least one or more of the urinary oxygen tension and a urine flow rate, and
   the program calculates the renal status index on the basis of the urinary oxygen tension and the urine flow rate, by using a predetermined algorithm.

5. The program according to claim 1 or 2, wherein

   the renal status index is an index related to an estimated value of a glomerular filtration rate,
   the urine information includes at least one or more of the urinary oxygen tension and a urine flow rate, and
   the program calculates the renal status index on the basis of the urinary oxygen tension and the urine flow rate, by using a predetermined algorithm.

6. The program according to any one of claims 1 to 5,
   outputting urine information and vital sign data together with the renal status index.

7. The program according to claim 1 or 2,

   acquiring circulatory dynamics information, and
   calculating the renal status index on the basis of the urine information and the circulatory dynamics information.

8. The program according to claim 7, wherein
   the circulatory dynamics information includes at least one or more of a mean blood pressure, a pulse pressure, a heart rate, blood oxygen saturation, and tissue oxygen saturation.

9. The program according to claim 7 or 8, wherein

   the renal status index is an index related to an estimated value of renal artery blood flow,
   the urine information includes at least one or more of the urinary oxygen tension and a urine flow rate,
   the circulatory dynamics information includes a mean blood pressure, a pulse pressure, and a heart rate, and
   the program calculates the renal status index on the basis of the urinary oxygen tension, the urine flow rate, the mean blood pressure, the pulse pressure, and the heart rate, by using a predetermined algorithm.

10. The program according to claim 7 or 8, wherein

    the renal status index is an index related to an estimated value of renal tissue oxygen tension,
    the urine information includes at least one or more of the urinary oxygen tension and a urine flow rate,
    the circulatory dynamics information includes a mean blood pressure, a pulse pressure, and a heart rate, and
    the program calculates the renal status index on the basis of the urinary oxygen tension, the urine flow rate, the mean blood pressure, the pulse pressure, and the heart rate, by using a predetermined algorithm.

11. The program according to claim 7 or 8, wherein

    the renal status index is an index related to an estimated value of a glomerular filtration rate,
    the urine information includes at least one or more of the urinary oxygen tension and a urine flow rate,
    the circulatory dynamics information includes a mean blood pressure, a pulse pressure, and a heart rate, and
    the program calculates the renal status index on the basis of the urinary oxygen tension, the urine flow rate, the mean blood pressure, the pulse pressure, and the heart rate, by using a predetermined algorithm.

12. The program according to any one of claims 7 to 11,
    outputting the urine information, the circulatory dynamics information, and vital sign data together with the renal status index.

13. The program according to any one of claims 1 to 12,

    repeatedly calculating the renal status index in time series, and
    outputting a newly calculated renal status index by using a relative value with a renal status index calculated in past.

14. The program according to any one of claims 1 to 13,

   acquiring information related to a drug scheduled to be administered,
   calculating a predicted value of the renal status index of a case where the drug administration is executed, and
   outputting the predicted value.

15. The program according to any one of claims 1 to 13,

   calculating a predicted value of the renal status index at a time after a predetermined time has elapsed is executed, and
   outputting the predicted value.

16. The program according to claim 15,

   calculating a difference between the predicted value and the renal status index at the time after the predetermined time has elapsed, and
   calculating a predicted value of a renal status index in a future by using the difference.

17. The program according to any one of claims 1 to 16,
   outputting a treatment guideline on the basis of the renal status index.

18. The program according to claim 17, wherein
   the treatment guideline is a treatment guideline intended to bring a relation between a urine flow rate and urinary oxygen tension into a predetermined status.

19. The program according to claim 17, wherein
   the treatment guideline is a treatment guideline intended to bring a renal status index into a predetermined value.

20. The program according to any one of claims 17 to 19,
   causing a drug administration device to administer a drug on the basis of the treatment guideline.

21. The program according to claim 20,

   repeatedly calculating the renal status index in time series, and
   changing an amount of a drug that the drug administration device is caused to administer on the basis of a change in the renal status index after the drug administration device administers the drug.

22. The program according to any one of claims 17 to 21,

   acquiring, in a case where a renal status index and a treatment guideline are input, a predicted value of a renal status index after drug administration based on the treatment guideline is executed, by using a trained model that outputs a renal status index after drug administration based on the treatment guideline is executed,
   calculating a difference between the predicted value and an actual renal status index after drug administration based on the treatment guideline is executed, and
   performing retraining of the trained model on the basis of the difference.

23. The program according to any one of claims 17 to 21,

   acquiring, in a case where a renal status index and a treatment guideline are input, a predicted value of a renal status index after drug administration based on the treatment guideline is executed, by using a trained model that outputs a renal status index after drug administration based on the treatment guideline is executed,
   calculating a difference between the predicted value and an actual renal status index after drug administration based on the treatment guideline is executed, and
   adjusting output data of the trained model on the basis of the difference.

24. An information processing method, wherein

a computer execute processing including
acquiring urine information including urinary oxygen tension,
calculating a renal status index on the basis of the urine information, and
outputting the renal status index.

25. An information processing apparatus comprising a control unit, wherein

the control unit
acquires urine information including urinary oxygen tension,
calculates a renal status index on the basis of the urine information, and
outputs the renal status index.

# FIG. 1

311
URINE SENSOR

10

31
URINE MEASUREMENT DEVICE

33
VITAL SIGN MONITOR

34
SYRINGE PUMP

35
INFUSION PUMP

17
ELECTRONIC MEDICAL RECORD SYSTEM

20
INFORMATION PROCESSING APPARATUS

21
CONTROL UNIT

22
MAIN STORAGE DEVICE

24
COMMUNICATION UNIT

25
DISPLAY UNIT

26
INPUT UNIT

23
AUXILIARY STORAGE DEVICE

# FIG. 2

```
            ( START )
                |
   +-------------------------------+  S501
   |   ACQUIRE URINE INFORMATION   |
   +-------------------------------+
                |
   +-------------------------------+  S502
   |     ACQUIRE CIRCULATORY       |
   |     DYNAMICS INFORMATION      |
   +-------------------------------+
                |
   +-------------------------------+  S503
   |  CALCULATE RENAL STATUS INDEX |
   +-------------------------------+
                |
   +-------------------------------+  S504
   |   ACQUIRE URINE INFORMATION   |
   +-------------------------------+
                |
   +-------------------------------+  S505
   |     ACQUIRE CIRCULATORY       |
   |     DYNAMICS INFORMATION      |
   +-------------------------------+
                |
   +-------------------------------+  S506
   |  CALCULATE RENAL STATUS INDEX |
   +-------------------------------+
                |
   +-------------------------------+  S507
   |  CALCULATE DIMENSIONLESS INDEX|
   +-------------------------------+
                |
   +-------------------------------+  S508
   |            DISPLAY            |
   +-------------------------------+
                |
           S509
       NO  < END? >
            |  YES
         ( END )
```

*FIG. 3*

201X/MM/DD (FRI) AM10:34
27.2°C/ 979hPa

RENAL STATUS
INDEX

# 84 /100

| URINARY OXYGEN TENSION | URINE FLOW RATE | MEAN BLOOD PRESSURE | HEART RATE |
|---|---|---|---|
| 21 | 0.8 | 112 | 68 |
| mmHg | mL/min | mmHg | /min |

(54kg/ 167cm/ M/ 59yr)

*FIG. 4*

201X/MM/DD (FRI) AM10:34
27.2°C/ 979hPa

RENAL STATUS
INDEX

# 18 /100

INDEX

50

TIME

| URINARY OXYGEN TENSION | URINE FLOW RATE | MEAN BLOOD PRESSURE | HEART RATE |
|---|---|---|---|
| 21 | 0.8 | 112 | 68 |
| mmHg | mL/min | mmHg | /min |

(54kg/ 167cm/ M/ 59yr)

## FIG. 5

201X/MM/DD (FRI) AM10:34
27.2°C/ 979hPa

RENAL STATUS
INDEX

**18** /100

INDEX

50

TIME

(54kg/ 167cm/ M/ 59yr)

## FIG. 6

201X/MM/DD (FRI) AM10:34
27.2°C/ 979hPa

| URINARY OXYGEN TENSION | URINE FLOW RATE | SpO2 |
|---|---|---|
| **18**mmHg | **0.7**mL/min | **93**mmHg |

| MEAN BLOOD PRESSURE | HEART RATE | FiO2 |
|---|---|---|
| **92**mmHg | **61**/min | **21**mmHg |

(54kg/ 167cm/ M/ 59yr)

## FIG. 7

761    71

201X/MM/DD (FRI) AM10:34
27.2°C/ 979hPa

CONDITION SELECTION

DATA AVERAGE SECTION

○ 10sec  ○ 12hrs
◉ 30sec  ○ 24hrs
○ 1 min  ○ 48hrs
○ 10min  ○ 72hrs
○ 30min
○ 1 hrs
○ 1 min

CALCULATION PARAMETER

◉ APPROXIMATION FORMULA
◉ Pearson r
○ DETERMINATION COEFFICIENT
○ AKI RISK VALUE
○ HYPOTENSION RISK VALUE

X-AXIS ITEM

○ URINARY OXYGEN TENSION
◉ URINE FLOW RATE
○ MEAN BLOOD PRESSURE
○ SpO2
○ HEART RATE

Y-AXIS ITEM

○ URINARY OXYGEN TENSION
○ URINE FLOW RATE
◉ MEAN BLOOD PRESSURE
○ SpO2
○ HEART RATE

(54kg/ 167cm/ M/ 59yr)

74

CALCULATION EXECUTION

$y = 0.89x + 13.1, pearson\ r = 0.78$

762

## FIG. 8

42

EXPLANATORY VARIABLE → RENAL STATUS MODEL → OBJECTIVE VARIABLE

# FIG. 9

EXPLANATORY VARIABLE → RENAL STATUS MODEL 42 → OBJECTIVE VARIABLE → TREATMENT GUIDELINE MODEL 44 → TREATMENT GUIDELINE

PATIENT INFORMATION

# FIG. 10

START

ACQUIRE URINE INFORMATION — S501

ACQUIRE CIRCULATORY DYNAMICS INFORMATION — S502

CALCULATE RENAL STATUS INDEX — S503

ACQUIRE URINE INFORMATION — S504

ACQUIRE CIRCULATORY DYNAMICS INFORMATION — S505

CALCULATE RENAL STATUS INDEX — S506

CALCULATE DIMENSIONLESS INDEX — S507

ACQUIRE TREATMENT GUIDELINE — S521

DISPLAY — S522

END? — S509

NO

YES

END

*FIG. 11*

72    721    78    71

201X/MM/DD (FRI) AM10:34
27.2°C/ 979hPa

RENAL STATUS
INDEX

PROPOSAL

**48** /100

● SYRINGE PUMP 1
ISOSORBIDE DINITRATE INJECTION 25mg 0.6mL/min

○ SYRINGE PUMP 2
NITROGLYCERIN INJECTION

(54kg/ 167cm/ M/ 59yr)

● INFUSION PUMP 1        ▶▶▶
RINGER'S SOLUTION  500mL   5mL/min

● INFUSION PUMP 2        ▶▶▶
NORADRENALINE INJECTION   0.5mL/min

74        771        772

*FIG. 12*

72    721    78    71

201X/MM/DD (FRI) AM10:34
27.2°C/ 979hPa

RENAL STATUS
INDEX

PROPOSAL

**26** /100

CONSIDER STOP OF
INFUSION PUMP 2
AND START OF SYRINGE
PUMP 1

● SYRINGE PUMP 1
ISOSORBIDE DINITRATE INJECTION 25mg 0.6mL/min

○ SYRINGE PUMP 2
NITROGLYCERIN INJECTION

(54kg/ 167cm/ M/ 59yr)

● INFUSION PUMP 1        ▶▶▶
RINGER'S SOLUTION  500mL   5mL/min

● INFUSION PUMP 2        ▶▶▶
NORADRENALINE INJECTION   0.5mL/min

74        771        772

## FIG. 13

EXPLANATORY VARIABLE → RENAL STATUS MODEL [42] → TIME-SERIES OBJECTIVE VARIABLE

## FIG. 14

201X/MM/DD (FRI) AM10:34
27.2℃/ 979hPa

RENAL STATUS INDEX

**84** /100

PREDICTED VALUE

**48**

◄ 6h ► 24

(54kg/ 167cm/ M/ 59yr)

## FIG. 15

## FIG. 16

# FIG. 17

START

ACQUIRE URINE INFORMATION S541

ACQUIRE CIRCULATORY DYNAMICS INFORMATION S542

ACQUIRE PATIENT INFORMATION S543

ACQUIRE DRUG SCHEDULED TO BE ADMINISTERED S544

ACQUIRE PREDICTIVE INDEX AFTER DRUG IS ADMINISTERED S545

S546 IS DRUG ADMINISTERED? NO

YES

TIMER S547

ACQUIRE MEASURED VALUE S548

CALCULATE DIFFERENCE S549

S550 EQUAL TO OR SMALLER THAN THRESHOLD VALUE? NO

CORRECT MODEL S551

YES

NO S552 END?

YES

END

# FIG. 18

## INTERNATIONAL SEARCH REPORT

| International application No. |
| --- |
| **PCT/JP2023/008084** |

**A. CLASSIFICATION OF SUBJECT MATTER**

*A61B 10/00*(2006.01)i; *A61B 5/026*(2006.01)i; *A61B 5/145*(2006.01)i; *A61B 5/20*(2006.01)i
FI: A61B10/00 U; A61B5/026 140; A61B5/145; A61B5/20

According to International Patent Classification (IPC) or to both national classification and IPC

**B. FIELDS SEARCHED**

Minimum documentation searched (classification system followed by classification symbols)

A61B10/00; A61B5/026; A61B5/145; A61B5/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | US 2020/0205718 A1 (SWSA MEDICAL VENTURES LLC) 02 July 2020 (2020-07-02) paragraphs [0095]-[0105] | 1-2, 4, 7-8, 12, 24-25 |
| Y | paragraphs [0095]-[0105] | 3, 5-6, 13-21 |
| A | paragraphs [0095]-[0105] | 9-11, 22-23 |
| Y | JP 2017-165772 A (MAYO FOUND. FOR MEDICAL EDUCATION & RESEARCH) 21 September 2017 (2017-09-21) paragraph [0169] | 3, 5-6, 13-21 |
| A | paragraph [0169] | 9-11, 22-23 |
| Y | JP 2021-106936 A (TERUMO CORP.) 29 July 2021 (2021-07-29) paragraphs [0264], [0346] | 13-21 |
| A | paragraphs [0264], [0346] | 9-11, 22-23 |
| Y | WO 2021/192971 A1 (TERUMO CORP.) 30 September 2021 (2021-09-30) paragraphs [0030]-[0087] | 14-21 |
| A | paragraphs [0030]-[0087] | 9-11, 22-23 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **07 April 2023** | **18 April 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/008084**

| Patent document cited in search report | | | Publication date (day/month/year) | Patent family member(s) | | | Publication date (day/month/year) |
|---|---|---|---|---|---|---|---|
| US | 2020/0205718 | A1 | 02 July 2020 | WO 2019/050984 A1 page 18, line 24 to page 23, line 3 | | | |
| JP | 2017-165772 | A | 21 September 2017 | US 2018/0327450 A1 paragraph [0275] CN 107496899 A paragraph [0271] | | | |
| JP | 2021-106936 | A | 29 July 2021 | US 2019/0150801 A1 paragraphs [0337], [0419] EP 3469982 A1 paragraphs [0247], [0326] CN 109152532 A paragraphs [0321], [0403] | | | |
| WO | 2021/192971 | A1 | 30 September 2021 | CN 115334977 A paragraphs [0044]-[0102] | | | |

Form PCT/ISA/210 (patent family annex) (January 2015)

**EP 4 491 132 A1**

**Patent documents cited in the description**

- JP 2021062073 A